# EUROPEAN PATENT APPLICATION

(11) **EP 4 102 217 A1**
(43) Date of publication of application: **14.12.2022**
(21) Application number: 22177674.3
(22) Date of filing: 07.06.2022
(51) Int. Cl.: G01N 27/12

(54) **HYDROGEN GAS SENSOR ASSEMBLY**

(30) Priority: 09.06.2021 US 202163208741 P
(71) Applicant: Carrier Corporation, Palm Beach Gardens, FL 33418 (US)
(72) Inventor: EVJU, Jon K., Minneapolis, MN 55438 (US); SANKARRAJ, Anand, Minneapolis, MN 55438 (US)
(74) Representative: Dehns

(57) **Abstract**

Disclosed is a hydrogen sensor assembly (10) comprising: a mounting portion (12); a sensor portion (14) disposed adjacent to the mounting portion; and at least one pedestal (18) connecting the sensor portion to the mounting portion wherein the sensor portion comprises a metal oxide semiconductor (62) having a hydrogen sensing surface (64) and the hydrogen sensing surface comprises a noble metal dopant.

## Description

### BACKGROUND

Gas sensors have been used in various applications such as process monitoring and control and safety monitoring. Because some gases can be flammable or explosive, gas detection sensors have also been used for leak detection where such gases are used or manufactured. Various types of sensors have been used or proposed, including but not limited to metal oxide semiconductor (MOS) sensors, pellistor (pelletized resistor) sensors, and electrochemical cells.

MOS sensors rely on interaction between gas test components with adsorbed oxygen on the metal oxide semiconductor surface. In the absence of the gas test components, the metal oxide semiconductor adsorbs atmospheric oxygen at the surface, and this adsorbed oxygen captures free electrons from the metal oxide semiconductor material, resulting in a measurable level of base resistance of the semiconductor at a relatively high level. Upon exposure to gas test components, the gas test component interacts with the adsorbed oxygen, causing it to release free electrons back to the semiconductor material, resulting in a measurable decrease in resistance that can be correlated with a measured level of test gas component.

In the case of top-doped metal oxide sensors, the exposed thin layer top dopant gives the metal oxide senor increased selectivity. The dopant's interaction with gas molecules on the sensor surface modulate changes in the metal oxide conductivity (or resistance) that are readily measured, and that are proportional to gas concentration.

Currently available MOS sensors for hydrogen are expensive to produce, can be poisoned by materials such as silicones, and can be affected by ambient conditions, such as temperature, pressure and humidity. An improved hydrogen sensor is desired.

### BRIEF DESCRIPTION

According to a first aspect of the invention, there is provided a hydrogen sensor assembly comprising: a mounting portion; and a sensor portion disposed adjacent to the mounting portion; wherein the sensor portion comprises a metal oxide semiconductor having a hydrogen sensing surface and the hydrogen sensing surface comprises a noble metal dopant. The hydrogen sensing surface may be free of metal dopants other than the noble metal dopant.

Optionally, the sensor portion includes a thermally conductive substrate, a heater, a temperature measurement device, and the metal oxide semiconductor is adjacent to the thermally conductive substrate. The sensor portion with the thermally conductive substrate, heater, and temperature measurement device maintains sensor temperature control to less than ±0.1 °C deviation from a set temperature.

Optionally, the metal oxide semiconductor includes tin (IV) oxide, titanium (IV) oxide, tungsten (VI) oxide, zinc (II) oxide, or a mixture of two or more of the foregoing and the noble metal dopant includes gold (Au), palladium (Pd), or a combination thereof.

Optionally, the metal oxide semiconductor includes tin (IV) oxide, and the noble metal dopant includes gold (Au).

Optionally, the noble metal dopant may have a thickness of 0.5 to 50 nanometers (nm).

Optionally, the hydrogen sensing surface has islands of the noble metal dopant.

Optionally, the sensor portion is maintained at a temperature of 110 to 250°C, or 140 to 230°C, or 190 to 220°C.

Optionally, the hydrogen sensor assembly may further include a pressure sensor, an ambient temperature sensor, a humidity sensor, or a combination thereof.

Optionally, the metal oxide semiconductor includes an additional metal dopant disposed within the metal oxide semiconductor.

According to a second aspect of the invention there is provided a method of measuring hydrogen gas concentration comprising contacting a hydrogen sensor with a gas and measuring the change in electrical resistance, wherein the hydrogen sensor comprises a comprises a metal oxide semiconductor having a hydrogen sensing surface and the hydrogen sensing surface comprises a metal dopant and the sensor is maintained at a temperature of 160 to 240°C. The hydrogen sensing surface may be free of metal dopants other than the noble metal dopant.

Optionally, the sensor is maintained at a temperature of 110 to 250°C, or 140 to 230°C, or 190 to 220°C.

The method according to the second aspect of the invention may comprise providing and/or using the sensor assembly and any features thereof as recited herein with reference to the first aspect of the invention.

### BRIEF DESCRIPTION OF THE DRAWINGS

The following descriptions should not be considered limiting in any way. Certain exemplary embodiments will now be described in greater detail by way of example and with reference to the accompanying drawings, in which like elements are numbered alike:
FIG. 1 is a perspective view of a hydrogen sensor assembly;
FIG. 2 is a perspective view of a sensor portion;
FIG. 3 is a cross section of the sensor portion; and
FIG. 4 is a graph of data described in the Examples.

### DETAILED DESCRIPTION

A detailed description of one or more embodiments of the disclosed apparatus and method are presented herein by way of exemplification and not limitation with reference to the Figures.

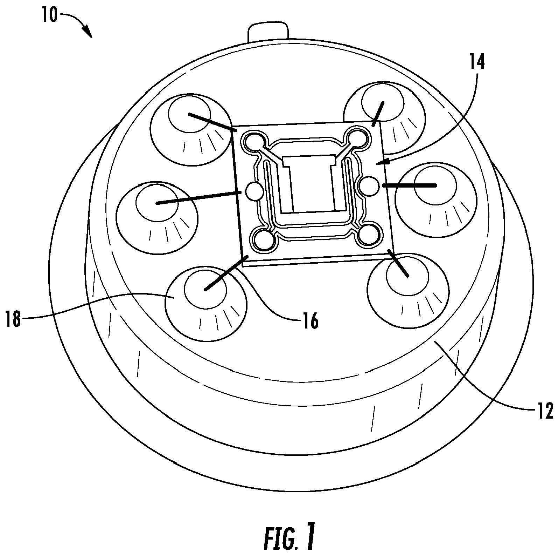

With reference now to the Figures, FIG. 1 illustrates a hydrogen sensor assembly 10. The sensor assembly 10 includes a mounting portion 12 and a sensor portion 14 connected to the mounting portion 12. The sensor portion 14 may be anchored to the mounting portion 12 by wires or ribbons 16. The wires or ribbons may be attached to the mounting portion at protrusions 18. The combination of wires/ribbons and protrusions allow the sensor portion to have limited physical contact with mounting portion which serves to improve thermal isolation of the sensor portion from the mounting portion.

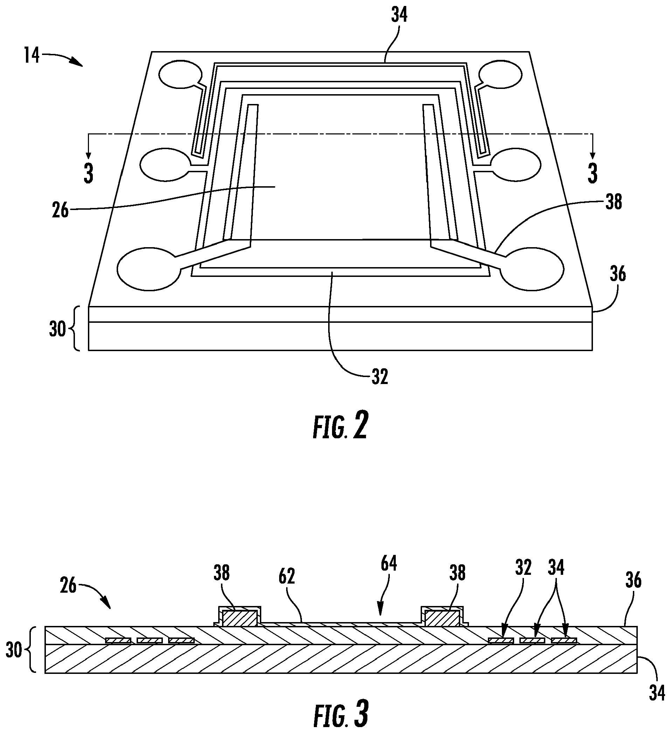

FIG. 2 shows the sensor portion 14 in more detail. The sensor portion 14 includes a sensor 26 adjacent to dielectric 36 which is disposed on substrate 30. Sensor 26 includes electrical connections 38. Substrate 30 may be thermally conductive. The sensor portion 14 may optionally include a heater 32 and a temperature measurement device 34. The temperature measurement device 34 and heater 32 may be part of a temperature control system that maintains the temperature of the sensor within a desired temperature range. The cross section indicated by line 3-3 is shown in FIG. 3.

The number and arrangement of protrusions 18 and the number and arrangement of the mounting portion 12 and the sensor portion 14 is not limited to the embodiment shown in FIG. 1. For example, the mounting portion 12 may be coupled to the sensor portion 14 only by two or more protrusions 18. The protrusions facilitate thermal isolation of the sensor 26 from the mounting portion by minimizing the physical structure and conduction area. Additionally, the use of two or more protrusions 18 to connect the mounting portion 12 and the sensor portion 14 stress isolates the sensor 26 from the mounting portion 12. Thermal isolation improves the energy efficiency, stability, selectivity and sensitivity of the sensor. Stress isolation improves performance of the sensor compared to a sensor that is not stress isolated.

Electrical connections 38 are part of an electrical circuit that includes a signal processor (not shown). The signal processor can be a voltmeter, ampere meter or Ohm meter, but in many cases comprises a potentiostatic circuit, voltage divider circuit, bridge circuit, microprocessor, electronic control unit (ECU), or similar electronic device with integrated voltage and or amperage measurement functions and also can apply a voltage bias between the electrical connections.

FIG. 3 is a cross section of sensor 26. As shown in FIG. 3, sensor 26, includes a metal oxide semiconductor 62 disposed on dielectric 36 which is part of substrate 30. The substrate may include more than one material and it is further contemplated that the substrate may include layers of multiple materials such as 34 which may be a thermally conductive layer. The metal oxide semiconductor 62 has a hydrogen sensing surface 64 that comprises the metal oxide semiconductor material and a noble metal dopant. Examples of metal oxide semiconductors include, but are not limited to, tin (IV) oxide, titanium (IV) oxide, tungsten (VI) oxide, zinc (II) oxide and mixtures of two or more of the foregoing. The noble metal dopant may be gold, palladium, or a combination thereof. In some embodiments the metal oxide semiconductor includes tin (IV) oxide and the metal dopant includes gold. The noble metal dopant may be present on the hydrogen sensing surface as islands, i.e., non-contiguous areas of noble metal dopant. The noble metal dopant may have a thickness of 0.5 to 50 nanometers. The noble metal dopant enhances the responsiveness and sensitivity of the metal oxide semiconductor to hydrogen. The metal oxide semiconductor may have an additional metal dopant within the metal oxide semiconductor. When present the interior metal dopant may be the same as or different from the surface dopant.

The sensor operates within a predetermined temperature range. An exemplary temperature range is 110 to 250°C. The temperature range may be 140 to 230°C, or 190 to 220°C. Due to the fast feedback on substrate temperature and its good heat conductivity, the sensor 26 maintains temperature control to less than ±0.1 °C deviation from a set temperature, even as the environment around the sensor element varies from -55 °C to + 85 °C.

Sensor 26 has excellent stability and is not readily poisoned by silicones when operated at lower temperatures, such as 110 to 170 °C. As mentioned above, sensor 26 operates at temperatures of 110 to 250°C which is markedly lower than other hydrogen sensors which are currently available. Sensor 26 is not affected by ambient or historic heat or humidity. The sensor 26 can operate with low power consumption allowing personal and fixed applications. Additionally, the sensor 26 can be readily mass produced. The foregoing features meet a need in the art for a cost effective hydrogen sensor.

Deposition of the metal oxide semiconductor onto the substrate can be performed using chemical vapor deposition or physical deposition techniques such as sputtering, or combinations of these. Alternatively, the metal oxide can be grown using solution-based epitaxy techniques such as sol-gel processing.

Deposition of the metal dopant onto the metal oxide semiconductor can be performed using thermal deposition techniques such as sputtering, physical vapor deposition, chemical vapor deposition, thermal spray, or atomic layer deposition. The surface of the metal oxide semiconductor can be covered with a template to form islands of the metal dopant. The metal oxide semiconductor may be annealed in air prior to application of the dopant, after application of the dopant, or both. Exemplary annealing conditions include 250 to 450°C for 10 to 90 minutes.

The sensor 26 may detect hydrogen in amounts greater than or equal to 100 parts per billion (ppb). The sensor may have a detection range corresponding to an environmental lower flammability level such as 100 ppb to 4 volume percent (vol%). The sensor 26 has minimal cross reactivity with hydrogen sulfide.

The sensor 26 can be stepped through a series of specific temperatures where sensor sensitivity to other gases (i.e. hydrogen sulfide) can be modulated and the amounts of both gases can be estimated.

The hydrogen sensor can be operated as part of an array of sensors, as a separate sensor element or as a separate sensor in a separate detector housing adjacent to other gas sensors and detectors.

The hydrogen sensor can be operated with an adjacent environmental sensor to aid algorithms for correcting for fluctuations in ambient pressure, temperature, and humidity.

Other sensor components including but not limited housings, mounting hardware, gas flow conduits, fluid chambers are not shown in the figures, but can be incorporated into the sensor by the skilled person.

Additional disclosure is provided in the following Examples:

### EXAMPLES

Five sensors were made, each of which had a tin oxide semiconductor body and a hydrogen sensing surface of tin oxide doped with gold. The sensors were annealed after doping according to the conditions shown in the Table below.

| | Time (minutes) | Temperature (°C) |
|---|---|---|
| A | 90 | 300 |
| B | 10 | 400 |
| c | 15 | 400 |
| D | 30 | 400 |
| E | 90 | 400 |

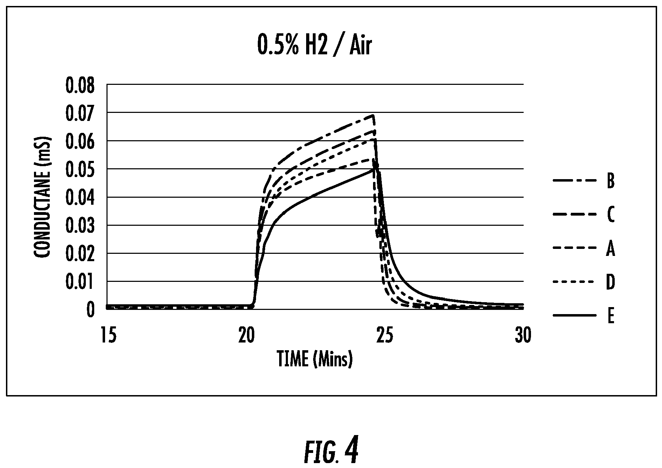

The sensors were evaluated for conductance changes in the presence of 0.5 volume percent of hydrogen in air at ambient temperature and pressure. The results are shown in FIG. 4. A comparative sensor having a tin oxide semiconductor body and a hydrogen sensing surface of tin oxide doped with gold and copper was also made. The comparative sensor with copper and gold doping showed negligible conductance under the same conditions.

The terminology used herein is for the purpose of describing particular embodiments only and is not intended to be limiting of the present disclosure. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, element components, and/or groups thereof. Furthermore, the terms "comprises" and/or "comprising," as well as the terms "includes" and/or "including," support embodiments which do not incorporate elements other than those described.

While the present disclosure has been described with reference to an exemplary embodiment or embodiments, it will be understood by those skilled in the art that various changes may be made and equivalents may be substituted for elements thereof without departing from the scope of the invention as set out in the appended claims. In addition, many modifications may be made to adapt a particular situation or material to the teachings of the invention without departing from the essential scope thereof. Therefore, it is intended that the invention not be limited to the particular embodiment disclosed as the best mode contemplated for carrying out this invention, but that the invention will include all embodiments falling within the scope of the claims.

## Claims

1. A hydrogen sensor assembly (10) comprising:
a mounting portion (12);
a sensor portion (14) disposed adjacent to the mounting portion; and
wherein the sensor portion comprises a metal oxide semiconductor (62) having a hydrogen sensing surface (64) and the hydrogen sensing surface comprises a noble metal dopant.

2. The hydrogen sensor assembly (10) of claim 1, wherein the hydrogen sensing surface (64) is free of metal dopants other than the noble metal dopant.

3. The hydrogen sensor assembly (10) of claim 1 or 2, wherein the sensor portion (14) comprises a thermally conductive substrate (30), a heater (32) and a temperature measurement device (34) and the metal oxide semiconductor (62) is adjacent to the thermally conductive substrate; optionally wherein the sensor portion (14) maintains sensor temperature control to less than ±0.1 °C deviation from a set temperature.

4. The hydrogen sensor assembly (10) of any preceding claim, wherein the metal oxide semiconductor (62) comprises tin (IV) oxide, titanium (IV) oxide, tungsten (VI) oxide, zinc (II) oxide, or a mixture of two or more of the foregoing and the noble metal dopant comprises gold, palladium, or a combination of gold and palladium.

5. The hydrogen sensor assembly (10) of any preceding claim, wherein the metal oxide semiconductor (62) comprises tin (IV) oxide and the noble metal dopant comprises gold.

6. The hydrogen sensor assembly (10) of any preceding claim, wherein the noble metal dopant has a thickness of 0.5 to 50 nanometers.

7. The hydrogen sensor assembly of any preceding claim, wherein the hydrogen sensing surface (64) comprises islands of the noble metal dopant.

8. The hydrogen sensor assembly (10) of any preceding claim, wherein the sensor (26) is maintained at a temperature of 110 to 250°C during operation; and preferably,
wherein the sensor (26) is maintained at a temperature of 140 to 230°C during operation; and more preferably,
wherein the sensor (26) is maintained at a temperature of 190 to 220°C during operation.

9. The hydrogen sensor assembly (10) of any preceding claim, wherein the sensor (26) has a detection range matching the concentration of the lower flammability levels for hydrogen.

10. The hydrogen sensor assembly (10) of any preceding claim, further comprising a pressure sensor, an ambient temperature sensor, a humidity sensor, or a combination thereof.

11. The hydrogen sensor assembly (10) of any preceding claim, wherein the metal oxide semiconductor (62) comprises an additional metal dopant disposed within the metal oxide semiconductor.

12. A method of measuring hydrogen gas concentration comprising contacting a hydrogen sensor (26) with a gas and measuring the change in electrical resistance, wherein the hydrogen sensor comprises a metal oxide semiconductor (64) having a hydrogen sensing surface (62) and the hydrogen sensing surface comprises a metal dopant and the sensor is maintained at a temperature of 110 to 250°C.

13. The method of claim 12, wherein the hydrogen sensing surface (62) is free of metal dopants other than the noble metal dopant.

14. The method of claim 12 or 13, wherein the sensor (26) has a detection range matching the concentrations of the lower flammability levels.

15. The method of any of claims 12-14, wherein the sensor (26) is maintained at a temperature of 140 to 230°C; and optionally
wherein the sensor (26) is maintained at a temperature of 190 to 220°C.
